# EUROPEAN PATENT SPECIFICATION

(11) **EP 2 399 624 B1**
(45) Date of publication and mention of the grant of the patent: **07.10.2015**
(21) Application number: 10006421.1
(22) Date of filing: 21.06.2010
(51) Int. Cl.: A61M 5/158, A61M 5/00

(54) **Device for inserting a soft member into the tissue of the body of a patient**
Vorrichtung zur Einführung eines weichen Elements in das Körpergewebe eines Patienten
Dispositif pour insérer un élément souple dans le tissu corporel d'un patient

(43) Date of publication of application: 28.12.2011
(73) Proprietor: F.Hoffmann-La Roche AG, 4070 Basel (CH); Roche Diagnostics GmbH, 68305 Mannheim (DE)
(72) Inventor: Teutsch, David, 3251 Wengi (CH); Michel, Philipp, 3052 Zollikofen (CH); Huwiler, Christoph, 6340 Baar (CH); Kaufmann, Heiner, 3008 Bern (CH)
(74) Representative: Rentsch Partner AG

(56) References cited:
- EP-A1- 1 764 125
- EP-A1- 1 970 091
- WO-A1-2007/140631
- US-A1- 2004 158 207
- US-A1- 2007 093 754
- US-A1- 2008 319 414

## Description

The present invention relates to a device for inserting a soft member into the tissue of the body of a patient and to a method of inserting a soft member into the tissue of the body of a patient by using said device in accordance with the preambles of the independent claims.

For patients with a regular requirement for a medicament that can be administered by direct delivery into the body tissue or into the blood stream, for example certain groups of patients suffering from pain or patients with type I and type II diabetes, it can be useful to supply the body with the required quantity of medicament in liquid form via an infusion set with a cannula that is introduced at a suitable location into the body and that remains there over a longer period. Efforts are also increasingly being made to monitor certain medical parameters of a patient, for example the blood sugar value, continuously over quite a long period of time. For this purpose, a sensor arrangement is placed on the patient's body, with a suitable sensor that passes through the skin into the patient's body.

In order to keep irritation of the application site during the application period at a minimum, it is desirable that the cannula or sensor which is inserted into the body is designed as a soft elements. i.e. is designed as an element which has a certain flexibility so that it can follow certain movements of the body tissue. However, due to this structural flexibility, such cannulas and sensors require for their introduction into the body a guiding element, which provides a puncturing tip and stiffens them during the introduction movement.

Furthermore, since the infusion set or sensor has to be changed at regular intervals, for example every three days, in order to avoid infections, and since in outpatient treatment, for example in the case of diabetics, this is often done by the patients themselves and, on account of the introduction of the cannula or sensor into the skin, is associated with a certain amount of pain, it is important that such infusion sets and sensor arrangements can be applied easily and safely.

Still furthermore, after the introduction of the cannula or sensor into the body, the guiding element must be removed and disposed off in a save manner, since it is contaminated and a contact with other persons could result in the transfer of diseases, in particular in case of an accidental piercing of the skin of the person with the contaminated puncturing tip of the guiding element.

US 2002/0022855 A1 discloses insertion devices for infusion sets with soft cannulas that are stiffened by a guiding needle, by means of which the infusion set can be placed onto the application site by the force of a pre-tensioned spring so that the guiding needle with the cannula abruptly penetrates into the tissue of the patient. After application of the infusion set, the insertion device and the guiding needle are removed from the infusion set, either together or separately.

EP 1 970 091 A1 discloses an insertion head with a soft cannula that is stiffened by a guiding needle. The insertion head can be applied by hand or by a suitable insertion device also disclosed in EP 1 970 091 A1, by means of which the insertion head is placed onto the application site by the force of a pre-tensioned spring so that the guiding needle with the cannula abruptly penetrates into the tissue of the patient. In case the insertion head is applied by hand, after the application, the guiding needle is removed by gripping a holder connected to it and by pulling this holder in the opposite direction of the inserting direction of the cannula, thereby removing the guiding needle from the soft cannula. After the guiding needle is pulled out of the cannula, it is pivoted in a protected position within a housing forming the holder. In case the insertion head is applied with an insertion device, after the application either in a first step the insertion device is removed from the application site and after that in a second step the guiding needle is removed in the way described before or, in case a specific insertion device is used for applying the insertion head, in a first step the insertion device is removed together with the guiding needle and in a second step the guiding needle is released from the insertion device and after that is pivoted into a protected position within the housing forming the holder.

While with the disclosed insertion devices the introduction of the soft cannula of the infusion set into the tissue of the body is considerable easy and safe, the removal of the guiding needle needs a certain sleight of hand and may cause irritation at the puncture site by forces exerted onto the inserted cannula if not performed properly. Furthermore, the removal of the guiding needle is associated with a certain risk of injury, since after its removal from the infusion set it is uncovered and must be handled with care.

The US 2008/08319414 A1 discloses an insertion apparatus for a medical system, such as an ambulatory infusion device, having a cradle unit that is adhesively fixed to a patient's skin. Further disclosed is an inserting device in which an insertion member in form of a soft cannula and a guiding needle (penetrating member) are reliably fixed. The inserting device is temporarily connected with the cradle unit via a releasable snap fit. The inserting device further comprises a spring driven drive mechanism for inserting the insertion member with a linear insertion motion into the patient's skin via a corresponding aperture in the cradle unit and subsequently retracting the guiding needle, while the soft cannula establishes a snap-fit connection with the cradle unit. An infusion set with a soft cannula and an inserting device that is designed similar to the US 2008/08319414 A1 is disclosed in the US 2004/0158207 A1.

The WO 2007/140631 A1, the EP 1970091 A1, and the EP 1764125 A1 each disclose an infusion device with an insertion member and corresponding insertion devices for inserting the insertion member into the patient's skin in a linear insertion motion. In an initial position, the insertion member is arranged parallel to a contact surface which, during the insertion process, is adhesively attached to the patient's skin. Prior to the insertion, the insertion member is pivoted into a position where it projects from the contact surface.

An application by hand in any case has the disadvantage that it needs certain skills and, since it is accompanied by a certain pain, costs the patient quite an effort.

Therefore, the object of the present invention is to make available a device for inserting a soft member into the tissue of the body of a patient and a method of inserting a soft member into the tissue of the body of a patient which do not have, or at least partially avoid, the disadvantages of the prior art.

This object is achieved by the device and by the method according to the independent claims.

Accordingly, a first aspect of the invention concerns a device for inserting a soft member into the tissue of the body of a patient.

The device comprises a housing having at least one contact face for placing the device onto the skin of the body of the patient.

Furthermore, it comprises an insertion arrangement which is movably arranged within the housing and provides the soft member to be inserted into the tissue. The soft member is an element which has a certain flexibility so that, in the inserted state, it can follow certain movements of the body tissue. The insertion arrangement furthermore provides a guiding member for temporarily stiffening the soft member during introduction into the tissue. This guiding member also forms a puncturing tip for puncturing the skin of the body of the patient when inserting the soft member with its help into the tissue.

The device furthermore comprises drive means for effecting an insertion movement of the insertion arrangement from a first position, in which the puncturing tip of the guiding member is set back relative to the contact face, into a second position, in which said puncturing tip protrudes beyond the contact face of the housing, so that, when the insertion movement is performed while the device is placed with its contact face on the body of the patient, the soft member together with the guiding member is inserted into the tissue of the body.

Furthermore the device comprises drive means for effecting, after the insertion movement of the insertion arrangement has been performed, a retracting movement of the guiding member from the second position into a third position, in which its puncturing tip is set back relative to the contact face of the housing, so that, when the retracting movement is performed while the soft member is held in the second position, the guiding member is separated from the soft member.

The drive means for effecting the insertion movement can be the same or can be different drive means than the drive means for effecting the retracting movement.

Furthermore, the device is designed in such a manner that after the insertion arrangement has reached the second position at the end of the insertion movement, automatically the retraction movement of the guiding member is performed. Thus, the device is designed in such a manner that, upon an activation of the drive means for the insertion movement, the insertion movement of the insertion arrangement is performed and after that automatically the drive means for the retracting movement are activated and the retraction movement of the guiding member is performed.

By means of the device according to the invention the insertion of a soft member, like e.g. a soft cannula, into the tissue of the body of a patient becomes easy and safe even for unskilled persons and the risk of an irritation of the puncture site due to an unskilled removal of the guiding member as well as the risk of an unintended contact with the contaminated guiding member or even an injury is virtually eliminated.

In a preferred embodiment of the device, the soft member is a soft cannula and the guiding member is a guiding needle extending through the fluid channel of the soft cannula for temporarily stiffening it during introduction into the tissue. The guiding needle protrudes with a free end thereof out of the soft cannula thereby forming the puncturing tip for puncturing the skin of the body of the patient when inserting the soft cannula into the tissue. The drive means in this case are designed in such a manner that, when during the retracting movement of the guiding needle from the second position into the third position the soft cannula is held in the second position, the guiding needle is pulled out of the fluid channel of the soft cannula. In such embodiments, which typically are employed to apply infusion sets for the infusion of a liquid medicament, like e.g. insulin, to the bodies of a patients, the advantages of the invention become particularly apparent.

In a further preferred embodiment, the device further comprises activating means for activating the drive means which are designed in such a manner that they can be actuated by the user with one hand. This considerably facilitates the operation of the device and furthermore permits its use for the application of soft members in body regions which are difficult to access or which are not accessible with both hands.

In still a further preferred embodiment of the device, the drive means of the device preferably comprise at least one energy source for providing driving energy for the insertion movement and/or for the retracting movement, like e.g. a pre-tensioned spring element, a pressurized gas reservoir, a battery, an accumulator and/or a pyrotechnical propelling charge.

Additionally or alternatively it is preferred that the drive means comprise at least one energy storing element for providing driving energy for the insertion movement and/or the retracting movement, which, before use of the device, must be charged with energy. Preferably, said at least one energy storing element comprises a spring element that can be pre-tensioned and/or a gas reservoir that can be pressurized. Such devices are comfortable in use and allow an insertion movement with defined insertion speed and insertion force.

In case of embodiments in which the device comprises at least one energy storing element, it is preferred that the device further comprises manual energy charging means, which can be actuated by hand to transfer power generated by the muscles of the hand of a user to the at least one energy storing element. Preferably, these manual energy charging means are designed in such a manner that they can be actuated with one hand. Such embodiments have the advantage that they may be independent from foreign energy sources and, if so, can be operated everywhere and at any time.

By advantage, said manual energy charging means comprise a slide-shaped or button-shaped element that can be slid along or pushed into the housing or comprise two housing elements that can be pushed into or towards each other. The direction of sliding along the housing, pushing into the housing or pushing into or towards each other preferably is transverse, in particular perpendicular, to the direction of the insertion movement of the insertion arrangement. This considerably facilitates the integration of the manual energy charging means into the device and the operation of the device by the user.

In embodiments, in which the device comprises activating means which can be actuated by the user with one hand and furthermore comprises at least one energy storing element and manual energy charging means for charging said energy storing element, it is preferred that the activating means and the manual energy charging means are designed in such a manner that charging of the at least one energy storing element and activating of the driving means for effecting the insertion movement is effected through the same manual operation.

As already stated before, the drive means for effecting the insertion movement can be the same or can be different drive means than the drive means for effecting the retracting movement. Furthermore, the energy for the insertion movement and the energy for the retraction movement can commonly or separately be provided by one or by several energy sources of the same kind or of different kind, in particular by a combination of hand energy and pressurized gas and/or pre-loaded springs. For example, a pre-loaded spring might provide the insertion energy while the hand provides the retraction energy.

In accordance with the present invention, the insertion arrangement in the first position is oriented substantially perpendicular to a plane formed by the at least one contact face of the housing and the insertion movement is a linear movement along an axis perpendicular to said plane. By means of this, the insertion member can be inserted into the tissue of the body of a patient perpendicular to the surface of the skin of the body, which in most of the applications is preferred.

In an initial position, the insertion arrangement is oriented substantially parallel to said plane formed by the at least one contact face of the housing and before the insertion movement can be performed must be pivoted from said initial position into the first position. By this is becomes possible to provide devices according to the invention which in the original (non-used) state have small packaging dimensions.

If additionally the device further comprises activating means for activating the drive means, it is preferred that the device is designed in such a manner that, upon an actuating of the activating means, the insertion arrangement is automatically moved from the initial position into the first position. This facilitates the use of the device.

In still a further preferred embodiment of the device the insertion arrangement further comprises a base plate which is coupled to the soft member. This base plate carries, on its side which during the insertion movement is facing towards the puncturing tip of the guiding member, a layer of skin compatible glue for fixing the soft member to the skin of the body of the patient when inserting it into the tissue. The layer of skin compatible glue might be covered by a protection layer, like e.g. a protective film, which needs to be removed prior to performing the insertion movement of the insertion arrangement. Such embodiments are especially suitable for being formed by using commercially available infusion sets in which a soft cannula is through material bonding connected with a base plate carrying a layer of skin compatible glue.

In another preferred embodiment of the device, the contact face of the housing carries a layer of skin compatible glue for fixing it to the skin. Also in this embodiment, the layer of skin compatible glue might be covered by a protection layer, like e.g. a protective film, which needs to be removed prior to the use of the device. Said contact face is formed by a base plate which in a releasable manner is fixed to the rest of the housing. In this embodiment, the insertion arrangement comprises coupling means connected with the soft member for coupling the soft member to the base plate when the insertion arrangement is moved into the second position. In this embodiment, the base plate, which is used to fix the soft member to the body of the patient, is fixed with its layer of skin compatible glue at the desired application site to the skin of the body prior to the insertion movement of the insertion arrangement. This offers the advantage that the base plate does not need to be accelerated by the drive means during the insertion movement so that less driving energy is needed for effecting the insertion movement or the insertion movement can take place with a higher speed, resulting in a reduction of the pain associated with the piercing of the skin.

In that case it is furthermore preferred that the device is designed in such a manner that said base plate is automatically separated from the housing when the insertion arrangement is brought into the second position or latest when the guiding member is brought into the third position, so that the housing together with the guiding member received in it can without further handling steps be removed from the base plate and the soft member coupled to it. Through such a design, handling of the device is as easy and safe as possible.

In still a further embodiment, the device is designed in such a manner that, when the guiding member after the retracting movement is held in the housing in the third position and the housing has been removed from the soft member, the guiding member is contained within the housing in such a manner that its puncturing tip does not protrude out of the housing. Through this, the risk of contacting the contaminated guiding member and in particular the risk of an injury caused by the contaminated puncturing tip thereof is considerably reduced.

In still a further preferred embodiment, the device is designed in such a manner that, when the guiding member after the retracting movement is held in the housing in the third position and the housing has been removed from the soft member, the guiding member can not be separated from the housing or can be separated from the housing without contacting the guiding member or a holder element fixedly connected with the guiding member. The first alternative is preferred if the housing is designed as a disposable part so that the contaminated guiding member can be disposed off together with the housing. The second alternative is preferred if the housing is intended to be used several times and the guiding member for disposal is separated from the housing. In both cases the advantage is arrived at that for disposal of the contaminated guiding member only the housing needs to be contacted by hand, thus, the risk of a contact with the contaminated guiding member during or of an injury caused bit its puncturing tip is further reduced.

In the second alternative of the above preferred embodiment it is preferred that the device is formed by an inserting device which provides the housing and the drive means and by an insertion head, like e.g. an infusion set, which provides the insertion arrangement. The insertion head is received within the inserting device for being applied by it to the body of the patient. The inserting device is designed in such a manner that, after the application of the insertion head and the removal of the guiding member from its housing, it can receive another insertion head for applying it to the body of a patient. By means of this, the amount of waste generated in therapies employing the frequent insertion of soft members into the body of a patient can be kept at a minimum.

Preferably, the device according to the first aspect of the invention is used for applying an infusion set with a soft cannula to the body of a patient.

Disclosed is further a method of inserting a soft member into the tissue of the body of a patient by means of the device according to the first aspect of the invention. In a first step, the device is placed with its at least one contact face onto the skin of the body at the desired application site. In a second step, the drive means of the device are activated, thereby effecting the insertion movement of the insertion arrangement into the tissue of the body and after that the retracting movement of the guiding member. In a third step, the housing with the guiding member contained in it is separated from the soft member while the soft member remains inserted in the tissue of the body of the patient.

With the method according to the invention, a soft member can be inserted into the body even by unskilled or hampered persons in an easy and safe manner, without the risk of an irritation of the application site through an unskilled removal of the guiding member from the inserted soft member and without the risk of an unintended contact with the contaminated guiding member or of an injury.

In a preferred embodiment of the method, the soft member used in the method is a soft cannula and the guiding member used is a guiding needle, which, at the time the soft cannula is inserted into the tissue, extends through the fluid channel of the soft cannula.

Further preferred embodiments of the invention become apparent from the dependent claims and from the following description by way of the drawings. Therein show:
Fig. 1a a perspective view onto a device according to the invention in a first state;
Fig. 1b a perspective longitudinal section of the device of Fig. 1a;
Fig. 2a a perspective view of the device of Fig. 1a in a second state;
Fig. 2b a perspective longitudinal section of the device of Fig. 2a;
Fig. 3a a perspective view of the device of Fig. 1a in a third state;
Fig. 3b a perspective longitudinal section of the device of Fig. 3a;
Fig. 4a a perspective view of the device of Fig. 1a in a fourth state;
Fig. 4b a perspective longitudinal section of the device of Fig. 4a;
Fig. 5a a perspective view of the device of Fig. 1a in a fifth state;
Fig. 5b a perspective longitudinal section of the device of Fig. 5a;
Fig. 5c a cross section of the device of Fig. 5a;
Fig. 6 a perspective longitudinal section of the device of Fig. 1a in a sixth state; and
Fig. 7 a perspective longitudinal section of the device of Fig. 1a in a seventh state.

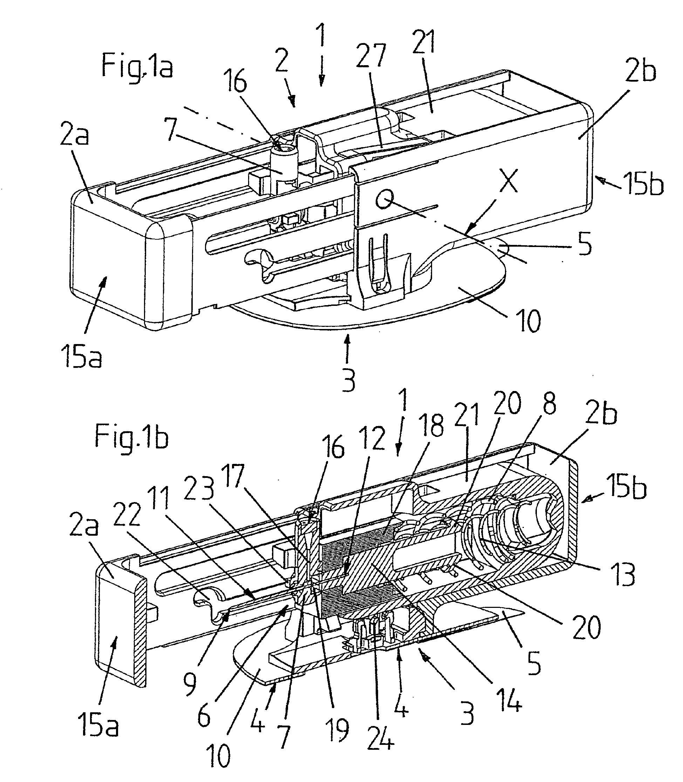

The Figures 1a and 1b show a device 1 according to the invention in a first state (original state), once in a perspective side view (Fig. 1a) and once in a perspective longitudinal section (Fig. 1b). As can be seen here, the device 1 comprises a housing 2 which substantially is formed by two housing elements 2a, 2a which for operating the device can be pushed into each other and a drive unit housing 21 which in the situation shown in these figures is arranged within the second housing element 2b. On its bottom side, the second housing element 2b carries a base plate 10 providing a contact face 3 for placing the device 1 onto the skin of the body of a patient. This contact face 3 carries a layer 4 of skin compatible glue for fixing the base plate 10 to the skin of the body, which layer 4 is covered by a removable protecting film 5 which before use of the device 1 must be removed.

Within the housing 2, 2a, 2b, a cannula arrangement 6 is arranged in a movable manner, which comprises a soft cannula 11 for being inserted into the tissue of the body of the patient and a guiding needle 12 extending through the fluid channel of said soft cannula 11 for temporarily stiffening it during introduction into the tissue. The guiding needle 12 at the free end of the cannula arrangement 6 protrudes out of the soft cannula 11, thereby forming a puncturing tip 9 for puncturing the skin of the body of the patient when inserting the soft cannula 11 with the help of the guiding needle 12 into the tissue. The soft cannula 11 at its end facing away from the puncturing tip 9 is fixedly connected through material bonding to an adapter housing 7 (holder element 7 according to the claims) providing a port 16 for connecting a catheter to the housing 7 and a fluid channel 17 extending inside the housing 7 between the soft cannula 11 and the port 16, for fluidically connecting the catheter with the soft cannula 11. The guiding needle 12 at its end facing away from its puncturing tip 9 protrudes through a septum 19 provided by the adapter housing 7 into a holder element 14 to which it is fixedly connected. The holder element 14 is in a slidable manner received in a guiding sleeve 18 which in turn in a slidable manner is guided inside the drive unit housing 21 which in a tiltable manner around an axis X is arranged inside the second housing element 2b. The soft cannula 11 and the adapter housing 7 are carried by the guiding needle 12. In this state, the cannula arrangement 6 is oriented substantially parallel to a plane formed by the contact face 3.

The device 1 furthermore comprises helical springs 8, 13 arranged in a coaxial manner within the drive unit housing 21, the function of which will in the course of the proceeding description of the drawings be described more into detail.

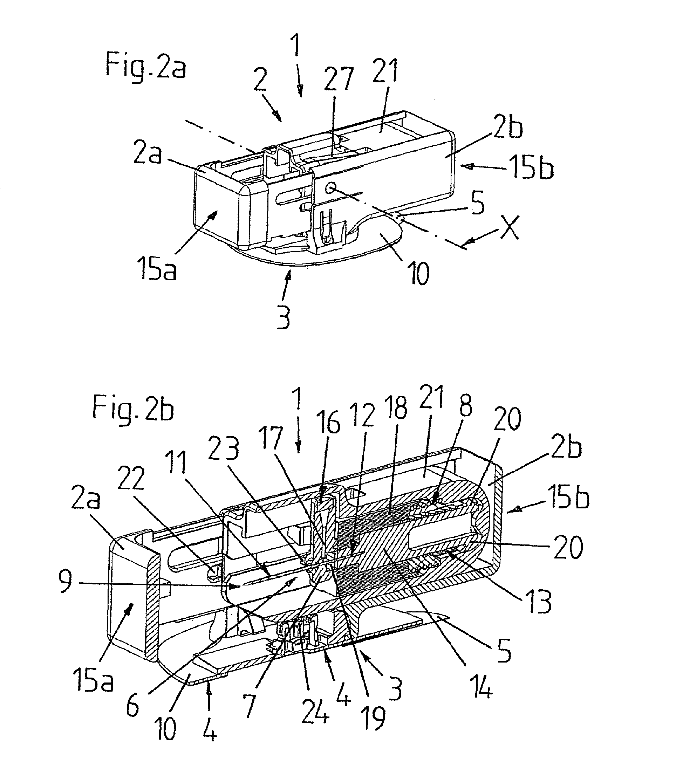

For operating the device 1, the front faces 15a, 15b (activating means 15a, 15b according to the claims) of the two housing elements 2a, 2b are contacted with the fingertips of the trigger finger and thumb of one hand of the operator and are pressed towards each other. By doing so, the two housing elements 2a, 2b are pushed into each other and the guiding sleeve 18, which carries the cannula arrangement 6, is pushed by the first housing element 2a into the drive unit housing 21 against the force of the two springs 8, 13, which both with one of their two ends abut against the guiding sleeve 18 and with their other end abut against an end of the drive unit housing 21. During this movement of the guiding sleeve 18, the holder element 14 travels through the centers of the springs 8, 13 and at the end, when both springs 8, 13 are completely pre-tensioned, snaps with resilient hook elements 20 formed at its end behind the end of the inner spring 13 which abuts against the end of the drive unit housing 21. At the same time, latching rockers 27 arranged at the drive unit housing 21 snap behind lugs 28 arranged at the guiding sleeve 18, thereby arresting the guiding sleeve 18 in a state in which the springs 8, 13 are pre-tensioned. This situation, in which the first housing element 2a has been pushed by about two thirds of its possible travel into the second housing element 2b, is shown in the Figures 2a and 2b, once in a perspective side view (Fig. 2a) and once in a perspective longitudinal section (Fig. 2b).

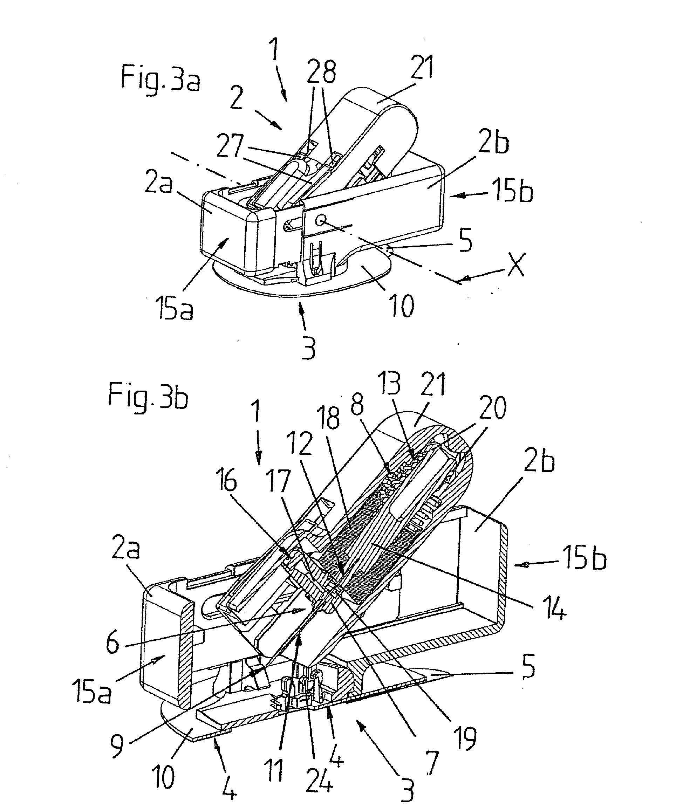

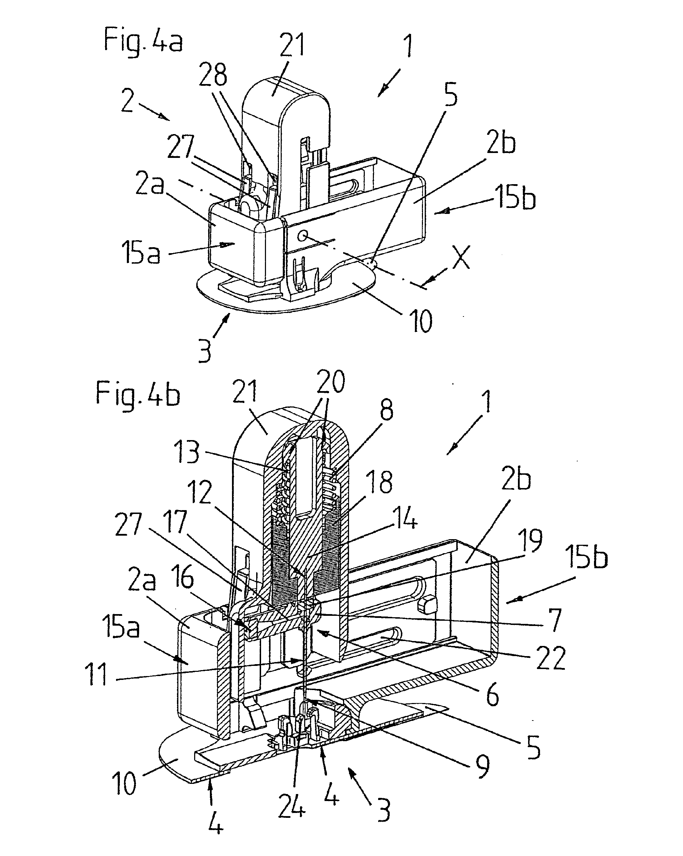

By further pushing the two housing elements 2a, 2b inside each other, the drive unit housing 21 with the cannula arrangement 6 carried by it is tilted around its tilting axis X from its initial position, in which the cannula arrangement 6 is oriented parallel to a plane formed by the contact face 3, over an intermediate position shown in the Figures 3a and 3b into its operating position (first position according to the claims) shown in the Figures 4a and 4b, in which the cannula arrangement 6 is oriented perpendicular to the plane formed by the contact face 3. In this situation, the puncturing tip 9 of the guiding needle 12 is set back relative to the contact face 3. The tilting movement of the drive unit housing 21 is effected by a pivot formed at the drive unit housing 21 which travels in a guiding slot 22 in the first housing element 2a. As is visible especially in Fig. 4a, in this state the housing elements 2a, 2b are almost fully pushed towards each other.

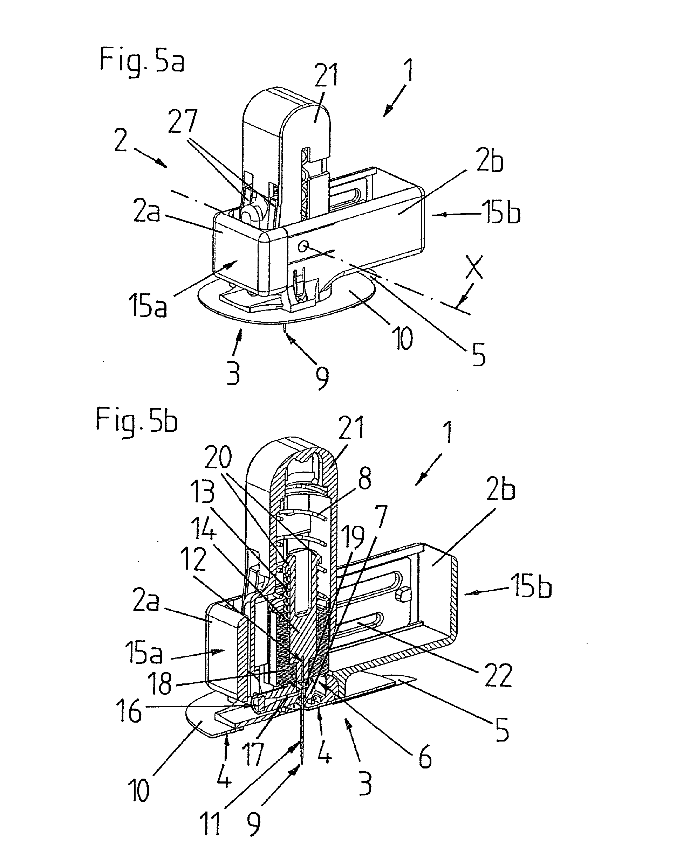

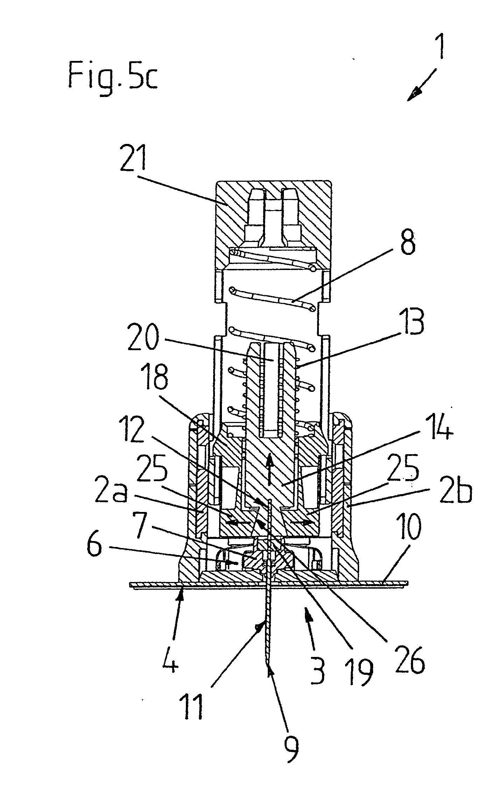

By completely pushing the two housing elements 2a, 2b towards each other, the latching rockers 27 are actuated, thereby releasing the lugs 28. The force of the pre-tensioned outer spring 8 now drives the guiding sleeve 18 and with it the cannula arrangement 6 in a direction perpendicular to the plane formed by the contact face 3 towards the base plate 10, where at the end of this movement (the insertion movement according to the claims) the adapter housing 7 with protrusions 23 formed at its outside snaps behind resilient hook elements 24 formed at the base plate 10 and thereby in a positive manner is coupled to the base plate 10. In this position (second position according to the claims), which is shown in the Figures 5a, 5b and 5c, the puncturing tip 9 and large portions of the guiding needle 12 as well as of the soft cannula 11 protrude beyond the contact face 3 of the base plate 10.

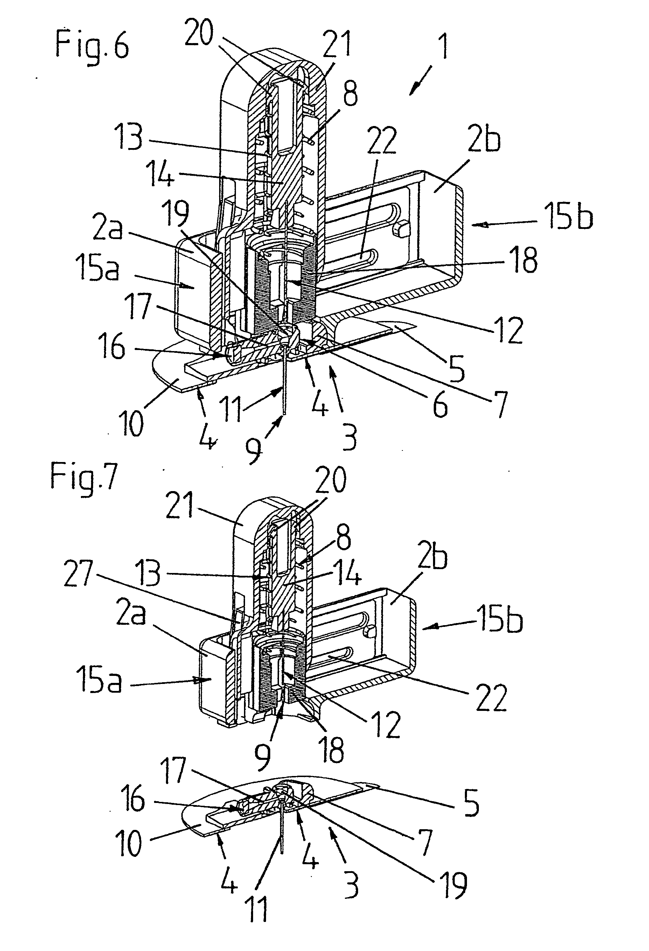

As can be seen in Fig. 5c, which shows a cross section of the device 1 in this state, the guiding sleeve 18 comprises two resilient hook elements 25, which abut with contact surfaces 26 that are inclined relative to the direction of the insertion movement against corresponding contact surfaces formed at the holder element 14 of the guiding needle 12. During the insertion movement, the hook elements 25 are radially blocked by guiding surfaces of the drive unit housing 21, so that during the insertion movement, the holder element 14 in a positive manner in movement direction is coupled to the guiding sleeve 18. In the end position of the insertion movement shown in the Figures 5a to 5c, the hook elements 25 are free to radially expand. In this situation, the holder element 14 is pulled upwards through the force of the inner spring 13, thereby radially expanding the hook elements 25 by its inclined contact surfaces (see the arrows in Fig. 5c) and pulling the guiding needle 12 out of the soft cannula 11 and out of the septum 19 of the adapter housing 7 (retracting movement according to the claims) into a position in which its puncturing tip 9 is set back relative to the contact face 3 (third position according to the claims). In this situation, which is shown in Fig. 6, the second housing element 2b can be detached from the base plate 10 and can, together with the guiding needle 12 contained therein, be removed from the base plated 10 without any risk that its puncturing tip can be contacted by hand and can cause an injury to persons. This situation is shown in Fig. 7.

While there are shown and described presently preferred embodiments of the invention, it is to be distinctly understood that the invention is not limited thereto but may be otherwise variously embodied and practiced within the scope of the following claims.

## Claims

1. Device (1) for inserting soft member (11) into the tissue of the body of a patient, comprising:
a) a housing (2, 2a, 2b, 10, 21) having at least one contact face (3) for placing the device (1) onto the skin of the body of the patient;
b) an insertion arrangement (6) movably arranged within the housing (2, 2a, 2b, 10, 21), comprising the soft member (11) to be inserted into the tissue and a guiding member (12) for temporarily stiffening the soft member (11) during introduction into the tissue, which guiding member (12) forms a puncturing tip (9) for puncturing the skin of the body of the patient when inserting the soft member (11) which is stiffened with it into the tissue; and
c) drive means (8, 13) for effecting an insertion movement of the insertion arrangement (6) from a first position into a second position,
wherein in the first position the insertion arrangement (6) is oriented substantially perpendicular to a plane formed by the at least one contact face (3) and the insertion movement is a linear movement along an axis perpendicular to said plane,
wherein the puncturing tip (9) is set back relative to the contact face (3) in the first position and protrudes beyond the contact face (3) in the second position, so that, when during the insertion movement the device (1) is placed with its contact face (3) on said body, the soft member (11) together with the guiding member (12) is inserted into the tissue of the body of the patient, and
the drive means (8, 13) are further for effecting, after the insertion movement of the insertion arrangement (6), a retracting movement of the guiding member (12) from the second position into a third position, in which its puncturing tip (9) is set back relative to the contact face (3), so that, when the soft member (11) is held in the second position, the guiding member (12) is separated from the soft member (11),
wherein the device (1) is designed in such a manner that after the insertion movement of the insertion arrangement (6) has been performed, automatically the retraction movement of the guiding member (12) is performed,
**characterized in that** in an initial position the insertion arrangement (6) is oriented substantially parallel to the plane formed by the at least one contact face (3) and, before the insertion movement can be performed, must be pivoted from the initial position into the first position by tilting a drive unit housing (21), the tilting being effected by a pivot formed at a drive unit housing (21) travelling in a guiding slot (22) of a first housing element (2a).

2. Device (1) according to claim 1, wherein the soft member (11) is a soft cannula (11) and the guiding member (1 2) is a guiding needle (12) extending through the fluid channel of the soft cannula (11) for temporarily stiffening it during introduction into the tissue,
wherein the guiding needle (12) at one end protrudes out of the soft cannula (11) thereby forming the puncturing tip (9) for puncturing the skin of the body of the patient when inserting the soft cannula (11) into the tissue
and wherein the drive means (8, 13) are designed in such a manner that, when during the retracting movement of the guiding needle (12) from the second position into a third position the soft cannula (11) is held in the second position, the guiding needle (12) is pulled out of the fluid channel of the soft cannula (11).

3. Device (1) according to one of the preceding claims, wherein the device (1) further comprises activating means (15a, 15b) for activating the drive means (8, 13) and wherein the activating means (15a, 15b) are designed in such a manner that they can be actuated by the user with one hand.

4. Device (1) according to one of the preceding claims, wherein the device (1) comprises separate drive means (8, 13) for the introduction movement (8) and for the retracting movement (13).

5. Device (1) according to claim 3, wherein the device (1) is designed in such a manner that, upon an actuating of the activating means (15a, 15b), the insertion arrangement (6) is automatically moved from the initial position into the first position.

6. Device (1) according to one of the preceding claims, wherein the insertion arrangement further comprises a base plate coupled to the soft member, which carries a layer of skin compatible glue for fixing the soft member to the skin of the body when inserting it into the tissue of the body of the patient.

7. Device (1) according to one of the claims 1 to 5, wherein the contact face (3) of the housing (2, 2a, 2b, 10, 21) carries a layer (4) of skin compatible glue for fixing it to the skin and is formed by a base plate (10) which in a releasable manner is fixed to the housing (2b), and wherein the insertion arrangement (6) comprises coupling means (23) connected with the soft member (11), by which the soft member (11) is coupled to the base plate (10) when the insertion arrangement (6) is moved into the second position.

8. Device (1) according to claim 7, wherein the device (1) is designed in such a manner that the base plate (10) is automatically separated from the housing (2b) when the insertion arrangement (6) is brought into the second position or when the guiding member (12) is brought into the third position so that rest of the housing (2, 2a, 2b, 21) together with the guiding member (12) can be removed from the base plate (10).

9. Device (1) according to one of the preceding claims, wherein the device (1) is designed in such a manner that, when the guiding member (12) after the retracting movement is held in the housing (2, 2a, 2b, 21) in the third position and the housing (2, 2a, 2b, 21) has been removed from the soft member (11), the guiding member (12) is contained within the housing (2, 2a, 2b, 21) in such a manner that its puncturing tip does not protrude out of the housing (2, 2a, 2b, 21).

10. Device (1) according to one of the preceding claims, wherein the device (1) is designed in such a manner that, when the guiding member (12) after the retracting movement is held in the housing (2, 2a, 2b, 21) in the third position and the housing (2, 2a, 2b, 21) has been removed from the soft member (11), the guiding member (12) can not be separated from the housing (2, 2a, 2b, 21) or can be separated from the housing (2, 2a, 2b, 21) without contacting the guiding member (12) or a holder element (14) fixedly connected with the guiding member (12).

11. Device (1) according to claim 10, wherein the device (1) is formed by an inserting device providing the housing and the drive means and an insertion head, in particular an infusion set, providing the insertion arrangement, which insertion head is received within the inserting device for being applied to the body of the patient by means of said inserting device, and wherein the inserting device is designed in such a manner that, after the application of the insertion head and the removal of the guiding member from its housing it can receive another insertion head for applying it to the body of a patient.

## Patentansprüche

1. Vorrichtung (1) zum Einführen eines weichen Elements (11) in das Körpergewebe eines Patienten, aufweisend:
a) ein Gehäuse (2, 2a, 2b, 10, 21) mit mindestens einer Kontaktfläche (3) zum Platzieren der Vorrichtung (1) auf der Haut des Körpers des Patienten;
b) eine Einführungsanordnung (6), die im Gehäuse (2, 2a, 2b, 10, 21) beweglich angeordnet ist, aufweisend das in das Gewebe einzuführende weiche Element (11) und ein Führungselement (12) zum vorübergehenden Versteifen des weichen Elements (11) während der Einführung in das Gewebe, wobei das Führungselement (12) eine Stechspitze (9) bildet zum Einstechen der Haut des Körpers des Patienten beim Einführen des weichen Elements (11), dass damit im Gewebe versteift wird; und
c) Antriebsmittel (8, 13) zur Ausführung einer Einführungsbewegung der Einführungsanordnung (6) aus einer ersten Position in eine zweite Position,
wobei die Einführungsanordnung (6) in der ersten Position im Wesentlichen senkrecht zu einer Ebene ausgerichtet ist, die durch die mindestens eine Kontaktfläche (3) gebildet ist, und die Einführungsbewegung eine lineare Bewegung entlang einer zu dieser Ebene senkrechten Achse ist,
wobei die Stechspitze (9) in der ersten Position relativ zur Kontaktfläche (3) zurückgesetzt ist und sich in der zweiten Position über die Kontaktfläche (3) hinaus erstreckt, sodass, wenn die Vorrichtung (1) während der Einführungsbewegung mit ihrer Kontaktfläche (3) auf dem Körper platziert wird, das weiche Element (11) zusammen mit dem Führungselement (12) in das Körpergewebe des Patienten eingeführt wird, und
wobei die Antriebsmittel (8, 13) weiter, nach der Einführungsbewegung der Einführungsanordnung (6), der Durchführung einer Rückzugsbewegung des Führungselements (12) aus der zweiten Position in eine dritte Position dienen in der ihre Stechspitze (9) relativ zur Kontaktfläche (3) zurückgesetzt ist, sodass, wenn das weiche Element (11) in der zweiten Position gehalten wird, das Führungselement (12) vom weichen Element (11) getrennt ist,
wobei die Vorrichtung (1) so konzipiert ist, dass nach dem Durchführen der Einführungsbewegung der Einführungsanordnung (6) automatisch die Rückzugsbewegung des Führungselements (12) ausgeführt wird,
**dadurch gekennzeichnet, dass** die Einführungsanordnung (6) in einer anfänglichen Position im Wesentlichen parallel zu der Ebene ausgerichtet ist, die durch die mindestens eine Kontaktfläche (3) gebildet ist, und, bevor die Einführungsbewegung durchgeführt werden kann, aus der anfänglichen Position in die erste Position geschwenkt werden muss, indem ein Antriebseinheits-Gehäuse (21) geneigt wird, wobei die Neigung durch einen an einem Antriebseinheits-Gehäuse (21) ausgebildeten Zapfen erreicht wird, der sich in einem Führungsschlitz (22) eines ersten Gehäuseelements (2a) bewegt.

2. Vorrichtung (1) nach Anspruch 1, wobei das weiche Element (11) eine Softkanüle (11) ist und das Führungselement (12) eine Führungsnadel (12) ist, die durch den Fluidkanal der Softkanüle (11) verläuft, um ihn während der Einführung in das Gewebe vorübergehend zu versteifen,
wobei die Führungsnadel (12) an einem Ende aus der Softkanüle (11) herausragt, sodass sie die Stechspitze (9) zum Einstechen in die Haut des Körpers des Patienten bildet, wenn die Softkanüle (11) in das Gewebe eingeführt wird,
und wobei die Antriebsmittel (8, 13) so konzipiert sind, dass, wenn die Softkanüle (11) während der Rückzugsbewegung der Führungsnadel (12) aus der zweiten Position in eine dritte Position in der zweiten Position gehalten wird, die Führungsnadel (12) aus dem Fluidkanal der Softkanüle (11) herausgezogen wird.

3. Vorrichtung (1) nach einem der vorhergehenden Ansprüche, wobei die Vorrichtung (1) weiter Aktivierungsmittel (15a, 15b) zum Aktivieren der Antriebsmittel (8, 13) aufweist, und wobei die Aktivierungsmittel (15a, 15b) so konzipiert sind, dass sie vom Benutzer mit einer Hand betätigt werden können.

4. Vorrichtung (1) nach einem der vorhergehenden Ansprüche, wobei die Vorrichtung (1) separate Antriebsmittel (8, 13) für die Einführungsbewegung (8) und für die Rückzugsbewegung (13) aufweist.

5. Vorrichtung (1) nach Anspruch 3, wobei die Vorrichtung (1) so konzipiert ist, dass beim Betätigen der Aktivierungsmittel (15a, 15b) die Einführungsanordnung (6) automatisch aus der anfänglichen Position in die erste Position bewegt wird.

6. Vorrichtung (1) nach einem der vorhergehenden Ansprüche, wobei die Einführungsanordnung weiter eine mit dem weichen Element gekoppelte Basisplatte aufweist, die eine Schicht hautverträglichen Klebstoffs trägt, um das weiche Element auf der Haut des Körpers zu fixieren, wenn es in das Körpergewebe des Patienten eingeführt wird.

7. Vorrichtung (1) nach einem der Ansprüche 1 bis 5, wobei die Kontaktfläche (3) des Gehäuses (2, 2a, 2b, 10, 21) eine Schicht (4) hautverträglichen Klebstoffs trägt, um sie auf der Haut zu fixieren, und wobei sie durch eine Basisplatte (10) gebildet ist, die lösbar am Gehäuse (2b) befestigt ist, und wobei die Einführungsanordnung (6) mit dem weichen Element (11) verbundene Kopplungsmittel (23) aufweist, durch die das weiche Element (11) an die Basisplatte (10) gekoppelt wird, wenn die Einführungsanordnung (6) in die zweite Position bewegt wird.

8. Vorrichtung (1) nach Anspruch 7, wobei die Vorrichtung (1) so konzipiert ist, dass die Basisplatte (10) automatisch vom Gehäuse (2b) getrennt wird, wenn die Einführungsanordnung (6) in die zweite Position gebracht wird oder wenn das Führungselement (12) in die dritte Position gebracht wird, sodass der Rest des Gehäuses (2, 2a, 2b, 21) zusammen mit dem Führungselement (12) von der Basisplatte (10) entfernt werden kann.

9. Vorrichtung (1) nach einem der vorhergehenden Ansprüche, wobei die Vorrichtung (1) so konzipiert ist, dass, wenn das Führungselement (12) nach der Rückzugsbewegung im Gehäuse (2, 2a, 2b, 21) in der dritten Position gehalten ist und nachdem das Gehäuse (2, 2a, 2b, 21) vom weichen Element (11) entfernt worden ist, das Führungselement (12) vom Gehäuse (2, 2a, 2b, 21) so umschlossen ist, dass seine Stechspitze nicht aus dem Gehäuse (2, 2a, 2b, 21) herausragt.

10. Vorrichtung (1) nach einem der vorhergehenden Ansprüche, wobei die Vorrichtung (1) so konzipiert ist, dass, wenn das Führungselement (12) nach der Rückzugsbewegung im Gehäuse (2, 2a, 2b, 21) in der dritten Position gehalten ist und nachdem das Gehäuse (2, 2a, 2b, 21) vom weichen Element (11) entfernt worden ist, das Führungselement (12) nicht vom Gehäuse (2, 2a, 2b, 21) getrennt werden kann, bzw. dass es vom Gehäuse (2, 2a, 2b, 21) getrennt werden kann, ohne dass es mit dem Führungselement (12) oder mit einem fest mit dem Führungselement (12) verbundenen Halterelement (14) in Kontakt gelangt.

11. Vorrichtung (1) nach Anspruch 10, wobei die Vorrichtung (1) durch eine Einführungsvorrichtung gebildet ist, die das Gehäuse und die Antriebsmittel und einen Insertionskopf, insbesondere ein Infusionsset, bereitstellt, der die Einführungsanordnung bereitstellt, wobei der Insertionskopf in der Einführungsvorrichtung aufgenommen ist, um mittels der Einführungsvorrichtung am Körper des Patienten angebracht zu werden, und wobei die Einführungsvorrichtung so konzipiert ist, dass nach dem Anbringen des Insertionskopfs und dem Entfernen des Führungselements aus seinem Gehäuse ein weiterer Insertionskopf zum Anbringen am Körper eines Patienten aufgenommen werden kann.

## Revendications

1. Dispositif (1) pour l'insertion d'un élément souple (11) dans le tissu du corps d'un patient, comprenant:
a) un boîtier (2, 2a, 2b, 10, 21) présentant au moins une face de contact (3) destinée à placer le dispositif (1) sur la peau du corps du patient;
b) un système d'insertion (6) arrangé de façon mobile à l'intérieur du boîtier (2, 2a, 2b, 10, 21), comprenant l'élément souple (11) à insérer dans le tissu et un élément de guidage (12) pour rigidifier temporairement l'élément souple (11) pendant l'introduction dans le tissu, ledit élément de guidage (12) formant une pointe de perforation (9) pour perforer la peau du corps du patient lors de l'insertion de l'élément souple (11) rigidifié par celle-ci dans le tissu; et
c) des moyens d'entraînement (8, 13) destinés à effectuer un mouvement d'insertion du système d'insertion (6) d'une première position dans une deuxième position,
dans lequel, dans la première position, le système d'insertion (6) est orienté sensiblement perpendiculairement à un plan formé par l'au moins une face de contact (3), et le mouvement d'insertion est un mouvement linéaire le long d'un axe perpendiculaire audit plan,
dans lequel la pointe de perforation (9) est en retrait par rapport à la face de contact (3) dans la première position et fait saillie au-delà de la face de contact (3) dans la deuxième position, de manière à ce que pendant le mouvement d'insertion, le dispositif (1) est placé avec sa face de contact (3) sur ledit corps, l'élément souple (11) ensemble avec l'élément de guidage (12) est inséré dans le tissu du corps du patient, et
les moyens d'entraînement (8, 13) sont en outre conçus pour effectuer, après le mouvement d'insertion du système d'insertion (6), un mouvement de rétraction de l'élément de guidage (12) de la deuxième position dans une troisième position, dans laquelle sa pointe de perforation (9) est mise en retrait par rapport à la face de contact (3), de manière à ce que, lorsque l'élément souple (11) est maintenu dans la deuxième position, l'élément de guidage (12) soit séparé de l'élément souple (11),
dans lequel le dispositif (1) est conçu de manière à ce que le mouvement de rétraction de l'élément de guidage (12) soit effectué automatiquement une fois que le mouvement d'insertion du système d'insertion (6) a été effectué,
**caractérisé en ce que** dans une position initiale, le système d'insertion (6) est orienté sensiblement parallèlement au plan formé par l'au moins une face de contact (3) et doit être pivoté de la position initiale dans la première position avant de pouvoir effectuer le mouvement d'insertion, par l'inclinaison d'un boîtier d'unité d'entraînement (21), l'inclinaison étant effectuée par un pivot formé au niveau d'un boîtier d'unité d'entraînement (21) se déplaçant dans une fente de guidage (22) d'un premier élément de boîtier (2a).

2. Dispositif (1) selon la revendication 1, dans lequel l'élément souple (11) est une canule souple (11) et l'élément de guidage (12) est une aiguille de guidage (12) s'étendant à travers le canal de fluide de la canule souple (11) pour la rigidifier temporairement pendant l'introduction dans le tissu,
dans lequel l'aiguille de guidage (12) fait saillie à une extrémité hors de la canule souple (11), formant ainsi la pointe de perforation (9) destinée à perforer la peau du corps du patient lors de l'insertion de la canule souple (11) dans le tissu,
et dans lequel les moyens d'entraînement (8, 13) sont conçus de telle façon que lorsque la canule souple (11) est maintenue dans la deuxième position pendant le mouvement de rétractation de l'aiguille de guidage (12) de la deuxième position dans une troisième position, l'aiguille de guidage (12) est tirée hors du canal de fluide de la canule souple (11).

3. Dispositif (1) selon l'une des revendications précédentes, dans lequel le dispositif (1) comprend en outre des moyens d'activation (15a, 15b) pour l'activation des moyens d'entraînement (8, 13), et dans lequel les moyens d'activation (15a, 15b) sont conçus de manière à pouvoir être actionnés d'une seule main par l'utilisateur.

4. Dispositif (1) selon l'une des revendications précédentes, dans lequel le dispositif (1) comprend des moyens d'entraînement (8, 13) séparés pour le mouvement d'introduction (8) et pour le mouvement de rétraction (13).

5. Dispositif (1) selon la revendication 3, dans lequel le dispositif (1) est conçu de telle façon que lors de l'actionnement des moyens d'activation (15a, 15b), le système d'insertion (6) est automatiquement déplacé de la position initiale dans la première position.

6. Dispositif (1) selon l'une des revendications précédentes, dans lequel le système d'insertion comprend en outre une plaque de base accouplée à l'élément souple, laquelle porte une couche de colle compatible avec la peau, pour fixer l'élément souple à la peau du corps lors de son insertion dans le tissu du corps du patient.

7. Dispositif (1) selon l'une des revendications 1 à 5, dans lequel la face de contact (3) du boîtier (2, 2a, 2b, 10, 21) porte une couche (4) de colle compatible avec la peau, pour fixer celle-ci sur la peau, et est formée par une plaque de base (10) fixée de façon amovible au boîtier (2b), et dans lequel le système d'insertion (6) comprend un moyen d'accouplement (23) connecté à l'élément souple (11), permettant d'accoupler l'élément souple (11) à la plaque de base (10) lors du déplacement du système d'insertion (6) dans la deuxième position.

8. Dispositif (1) selon la revendication 7, dans lequel le dispositif (1) est conçu de telle façon que la plaque de base (10) est automatiquement séparée du boîtier (2b) lorsque le système d'insertion (6) est mis dans la deuxième position ou lorsque l'élément de guidage (12) est mis dans la troisième position, de sorte que le reste du boîtier (2, 2a, 2b, 21) puisse être retiré de la plaque de base (10) conjointement avec l'élément de guidage (12).

9. Dispositif (1) selon l'une des revendications précédentes, dans lequel le dispositif (1) est conçu de telle façon que, lorsque l'élément de guidage (12) est maintenu dans le boîtier (2, 2a, 2b, 21) dans la troisième position suite au mouvement de rétraction, et que le boîtier (2, 2a, 2b, 21) a été retiré de l'élément souple (11), l'élément de guidage (12) est contenu à l'intérieur du boîtier (2, 2a, 2b, 21) de manière à ce que sa pointe de perforation ne fasse pas saillie hors du boîtier (2, 2a, 2b, 21).

10. Dispositif (1) selon l'une des revendications précédentes, dans lequel le dispositif (1) est conçu de telle façon que, lorsque l'élément de guidage (12) est maintenu dans le boîtier (2, 2a, 2b, 21) dans la troisième position suite au mouvement de rétractation, et que le boîtier (2, 2a, 2b, 21) a été retiré de l'élément souple (11), l'élément de guidage (12) ne puisse pas être séparé du boîtier (2, 2a, 2b, 21) ou puisse être séparé du boîtier (2, 2a, 2b, 21) sans toucher l'élément de guidage (12) ou un élément de maintien (14) relié fixement à l'élément de guidage (12).

11. Dispositif (1) selon la revendication 10, dans lequel le dispositif (1) est formé par un dispositif d'insertion fournissant le boîtier et les moyens d'entraînement et une tête d'insertion, en particulier un ensemble de perfusion, fournissant le système d'insertion, ladite tête d'insertion étant reçue à l'intérieur du dispositif d'insertion pour être appliquée sur le corps du patient au moyen dudit dispositif d'insertion, et dans lequel le dispositif d'insertion est conçu de façon à pouvoir recevoir une autre tête d'insertion destinée à être appliquée sur le corps d'un patient, suite à l'application de la tête d'insertion et au retrait de l'élément de guidage hors de son boîtier.
